# EUROPEAN PATENT APPLICATION

(11) **EP 3 406 731 A1**
(43) Date of publication of application: **28.11.2018**
(21) Application number: 17305596.3
(22) Date of filing: 22.05.2017
(51) Int. Cl.: C12Q 1/04, G01N 33/58, C12Q 1/16

(54) **METABOLIC LABELING OF BACTERIAL TEICHOIC ACIDS CELL WALL**

(71) Applicant: Commissariat à l'Energie Atomique et aux Energies Alternatives, 75015 Paris (FR); CENTRE NATIONAL DE LA RECHERCHE SCIENTIFIQUE, 75016 Paris (FR)
(72) Inventor: VERNET, Thierry, 38000 Grenoble (FR); WONG, Yung-Sing, 38400 Saint Martin d'Hères (FR); DI GUILMI, Anne-Marie, 38760 Saint Paul de Varces (FR)
(74) Representative: Gevers & Orès

(57) **Abstract**

The present invention provides a new method for the specific metabolic labeling of bacterial teichoic acids cell wall by modified choline and click chemistry, and its use in various applications such as bio-imaging, diagnostic, vaccination or bio-materials engineering.

## Description

The invention pertains to the field of bacterial labeling. The present invention provides a new method for the specific metabolic labeling of bacterial teichoic acids cell wall by modified choline and click chemistry, and its use in various applications such as bio-imaging, diagnostic, vaccination or bio-materials engineering.

The bacterial cell wall is composed by peptidoglycan (PG), *i.e.* a matrix of linear glycan chains of N-acetylmuramic acid and N-acetylglucosamine residues cross-linked via peptides strands made of Land D-amino acids. Labeling of the cell wall of bacteria is a very challenging task because this cell component displays essential functions (such as mechanical resistance, shape or attachment of other molecules) and, in particular, its assembly is the main Achilles' heel of bacteria targeted by beta-lactams, which encompass over 60% of the total antibiotics used today.

Given that the glycan polymers of the cell wall are not genetically encoded, they cannot be labelled by classic recombinant DNA techniques. Currently, immunolabeling is the main technique used to detect and localize cell surface components. This technique is convenient because it allows the labeling of both genetically and non-genetically encoded molecules but requires specific antibodies against each target molecule. The major disadvantage of this technique is that the cells have to be chemically fixed and permeabilized, a procedure that alters to a great extend the cell surface structure and prevents all kind of live cell imaging, a mandatory condition to decipher biological functions of molecules in a physiological environment.

In this context, a new field of research is emerging which consists in implementing chemical tools in cellular biology. These techniques have been developed for modifying cell surfaces with non-native synthetic compounds. The basic strategy to display small molecules of interest onto the surface of bacteria relies on their metabolic incorporation. These chemically modified molecules are added exogenously in the bacterial culture, taken up by the appropriate machinery and metabolically incorporated into the growing cell wall. Some of the polymers of the bacterial cell wall, such as peptidoglycan (Sadamoto et al., J. Am. Chem. Soc., 124:9018-9019, 2002), lipopolysaccharide (Liu et al., Proc. Natl. Acad. Sci. USA, 106:4207, 2009) or glycolipids (Backus et al., Nat. Chem. Biol., 7:228, 2011), have already been targeted by metabolic labeling.

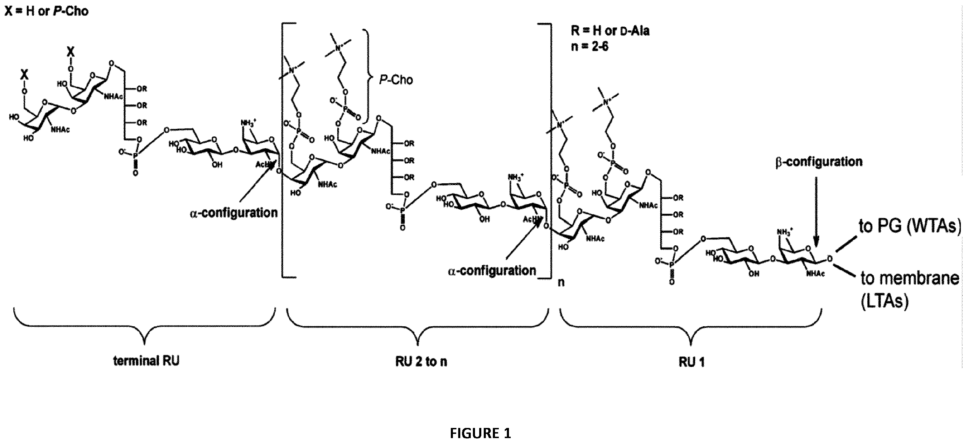

In the case of Gram-positive bacteria, the cell wall also contains Teichoic Acids (TAs). These glycopolymers are either attached to the peptidoglycan (wall teichoic acids, WTA) or anchored to the cytoplasmic membrane (lipoteichoic acid, LTA). TAs are complex polysaccharides made of a succession of 4-8 repeating units (as illustrated in **Figure 1****).** In pneumococci, one repeating unit contains AATGal*p*, Glc*p*, Rib-ol-5-P, and two Gal*p*NAc, both substituted in position O-6 with PhosphoCholine (P-Cho). All repeating units are α-1-linked, only the first is β-1-linked to the cell anchor. The hydroxyl groups of Ribol-5-P can be substituted in non-stoichiometric amounts by D-Ala. In LTAs, the cell anchor is a Glc*p*-diacylglycerol. In WTAs, the chain is attached to the MurNAc of the PG by way of a Gro*p*-ManNAc-GlcNAc*p* linkage unit.

TAs play important roles in host infection and participate to the regulation of cell morphology. In particular, it has been shown that bacteria depleted in TAs display shape defects and irregular wall thickness, indicating an intimate interplay between PG and TAs (Kawai et al., EMBO J., 30:4931, 2001; Santa Maria et al., Proc. Natl. Acad. Sci. USA, 111:12510, 2014). However, despite the essential roles of TA in physiopathological processes, knowledge on TA biosynthesis is hampered by the lack of appropriate methods to trace TA in live cells. Therefore, there is a need for new tools allowing the specific labeling and tracking of TA on live bacteria.

Here, the Inventors provide a new method for the specific labeling of pneumococcal TA by modified choline and click chemistry. Since this method is rapid, cheap and easy-to-use, it can be used for numerous applications, in particular for bio-imaging, diagnostic, vaccination and bio-materials engineering.

This invention results from the unexpected observation made by the Inventors that bacteria can metabolize a modified choline and incorporate it in the teichoic acid of the cell wall, allowing a bioorthogonal labeling reaction with a large variety of tag molecules.

### Method of labeling

In an aspect, the invention relates to a method of labeling a bacterium that is able to metabolize choline, said method comprising a step (i) of incubating the bacterium in a culture medium containing a modified choline which is metabolized by the bacterium, covalently associated to the TA into the cytoplasm, before being exported and integrated into the cell wall of the bacterium.

Unless defined otherwise, all technical and scientific terms used herein have the same meaning as commonly understood to one skilled in the art. For convenience, the meaning of certain terms and expressions employed in the specification, examples, and claims are provided.

In the invention, the expression "a bacterium that is able to metabolize choline" refers to a group of bacteria that possess the cell machinery required to import the choline present in the medium, to metabolize and to load the choline with teichoic acids.

In particular, the bacterium is a Gram-positive bacterium, which can be selected from the *Streptococcus* genus, or a Gram-negative bacterium, which can be selected from *Haemophilus* or *Neisseria* genera, and in particular from *S*. *pneumoniae, H. influenzae* and *Neisseria ssp..*

Indeed, it is known that the growth of *S*. *pneumoniae* is strictly dependent from choline and the phosphocholine (PCho), a derivative of choline, is used for the decoration of the teichoic acids. *H*. *influenzae* has PCho containing lipopolysaccharides, whereas *Neisseria ssp.* have PCho decorated lipopolysaccharides and proteinaceous pili. Both *H. influenzae* and some *Neisseria* species share with *S. pneumoniae* the same transporter (LicB) to import choline from the medium and they both expose PCho groups at their surface (Fan et al., Mol. Microbiol., 50:537, 2003; Serino et al., Mol. Microbiol., 43:437, 2002).

A bacterium able to metabolize choline can also be identified by screening the presence of specific enzymes, such as those encoded by the *lic* loci (*licA, licB, licC and licD1*, *licD2*), as for example the transporter encoded by the gene *licB* (Accession Number NP_358739.1), its homologous or orthologous genes in S. *pneumoniae.*

In an embodiment, the invention relates to the method as defined above, wherein the bacterium is selected from the *Streptococcus* genus, in particular from the species *S*. *pneumoniae* and *S*. *mitis,* more particularly from *S*. *pneumoniae.* In the invention, the choline dependency of pneumococcal growth is harnessed to enable the metabolic labeling with a modified choline.

In an embodiment, the invention relates to the method of labeling a bacterium as defined above, wherein the bacterium is selected from the *Haemophilus* genus, in particular from the *H. influenzae* species.

In an embodiment, the invention relates to the method of labeling a bacterium as defined above, wherein the bacterium is selected from the *Neisseria* genus, in particular from the *Neisseria ssp.,* more particularly from *N. lactamica* species.

In the invention, the expression "modified choline" refers to a choline that has been modified to integrate a chemical modification allowing the direct detection of the modified choline, *i.e.* via a direct labeling, or a chemical modification allowing a bioorthogonal reaction of the modified choline with a tag molecule, *i.e.* via an indirect labeling.

In the case of a direct labeling, the modified choline is chemically modified to incorporate a radioactive isotope.

In an embodiment, the invention relates to the method of labeling a bacterium as defined above, wherein the modified choline comprises a radioactive isotope, such as ³H or ¹⁵N.

In the case of an indirect labeling, the detection of the modified choline requires the addition of a tag molecule via a bioorthogonal reaction.

The expression "bioorthogonal reaction" is a generic and well-known expression that refers to a chemical reaction that is achieved inside or at the surface of a living cell without interfering with native biochemical processes.

In an embodiment, the invention relates to the method of labeling a bacterium as defined above, that further comprises a step (ii) of contacting the bacterium with a tag molecule to generate a binding reaction between the modified choline bound to the TA present in the cell wall of the bacterium and the tag molecule.

In a preferred embodiment, the invention relates to the method of labeling a bacterium as defined above, wherein, in step (ii), the binding reaction between the modified choline bound to the TA present in the cell wall of the bacterium and the tag molecule is made by a click chemistry reaction.

The term "click chemistry" is a generic term that encompasses a wide variety of chemical reactions between pairs of functional groups (or "clickable" reagents) that rapidly and selectively react with each other in aqueous conditions to form a stable conjugate. The click chemistry offers convenient, versatile and reliable two-steps coupling procedures of two molecules (*e.g*. "A" and "B") that are widely used in chemical biology, especially in the field of cell labeling, to generate bioorthogonal ligation reactions (for review, King et Wagner, Bioconjugate Chem., 25: 825, 2014).

The cell labeling requires reaction procedures that can be performed under physiological conditions (neutral pH, aqueous solution, ambient temperature) with low reactant concentrations to ensure non-toxic and low background labeling at reasonable time scales while still preserving the biological functions.

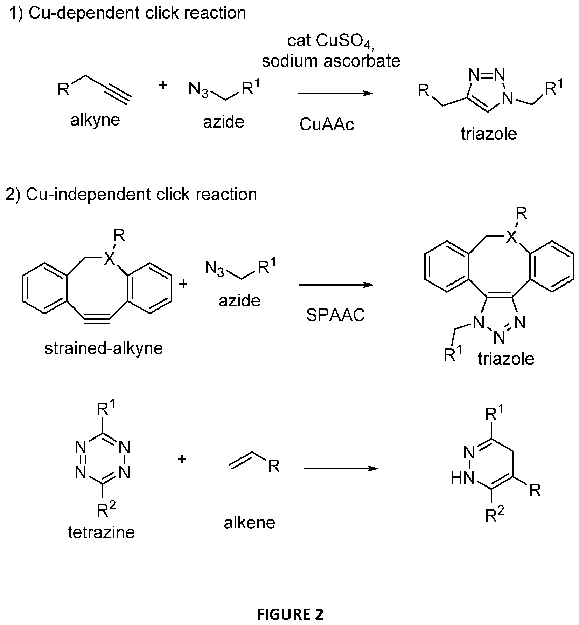

Click chemistry reactions is mainly categorized into two separate groups (as illustrated in **Figure 2****):**

### I. Copper (Cu(I)) - catalyzed reactions

This group of reactions mainly comprises, but is not limited to, the Cu(I)-catalyzed Azide-Alkyne (Copper-Catalyzed Azide-Alkyne Cycloaddition, CuAAC).

CuAAC reaction is the most prominent example of click chemistry. An azide-functionalized molecule A reacts with a terminal alkyne-functionalized molecule B thereby forming a stable conjugate A-B via a triazole moiety. The efficiency of a CuAAC reaction strongly depends on the presence of a metal catalyst such as copper (Cu) in the +1 oxidation state (Cu(I)). Different sources and reduction reagents are available. However, the Cu(II) salt CuSO₄ as copper source in combination with ascorbate as a reduction reagent has been recommended for most biomolecule labeling applications. The use of CuAAC reactions in live cells may be hampered by the toxicity of Cu(I) ions. This problem has been overcome by the use of Cu(I) chelating ligands such as tris(3-hydroxypropyltriazolylmethyl)amine (THPTA) that serve a dual purpose: (1) acceleration of the CuAAC reaction by maintaining the Cu(I) oxidation state and (2) protection of the biomolecule from oxidative damage.

### II. Copper free reactions

This group of reactions mainly comprises, but is not limited to, the following reactions:

### a. Strain-Promoted Azide-Alkyne (Strain-Promoted Azide-Alkyne Cycloaddition, SPAAC)

SPAAC reaction is a non-toxic labeling method. It relies on the use of strained cyclooctynes that possess a remarkably decreased activation energy in contrast to terminal Alkynes and thus do not require an exogenous catalyst. A number of structurally varied cyclooctyne derivatives (e.g. DIFO, BCN, DIBAC, DIBO, ADIBO) have been developed and they differ in terms of reaction kinetics and hydrophility.

### b. Alkene-Tetrazine

The Alkene-Tetrazine reaction is also a non-toxic labeling method that is ideally suited for *in vivo* cell labeling with high-speed and low concentration applications. A terminal or strained Alkene-functionalized molecule reacts with a Tetrazine-functionalized molecule B forming a stable conjugate A-B via dihydropyrazine moiety. A number of structurally varied alkene (*e.g*. TCO, vinyl, methylcyclopropene) and tetrazine derivatives (*e.g*. tetrazine, 6-Methyl-Tetrazine) have been developed and they differ in terms of reaction kinetics and hydrophility.

In an embodiment, the invention relates to the method of labeling a bacterium as defined above, wherein the modified choline comprises at least one reactive group X allowing the binding of the modified choline to the tag molecule, said at least one reactive group X being selected from the reactive groups consisting of an alkene group, an alkyne group, an azide group, a cyclopropenyl group and a diazirine group.

In an embodiment, the invention relates to the method of labeling a bacterium as defined above, wherein the modified choline corresponds to the formula (I): 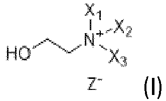 wherein the groups X₁, X₂ and X₃ are selected, independently from each other, from the reactive groups consisting of:
- an alkene group 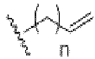 wherein n = 0 to 5, in particular n = 1, 2, 3, 4 or 5,
- an alkyne group 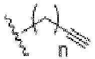 wherein n = 0 to 5, in particular n = 1, 2, 3, 4 or 5,
- an azide group 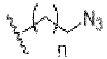 wherein n = 0 to 5, in particular n = 1, 2, 3, 4 or 5,
- a cyclopropenyl group 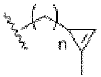 wherein n = 0 to 5, in particular n = 1, 2, 3, 4 or 5,
- a diazirine group wherein n = 0 to 5, in particular n = 1, 2, 3, 4 or 5,
and from a methyl group 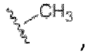 and
at least one, preferably two, more preferably three, of the reactive groups X₁, X₂ and X₃ is different from a methyl group, and
Z⁻ is a counterion, preferably selected from F⁻, Cl⁻, Br⁻, I⁻, Tosyl⁻, Mesyl⁻, Triflate⁻, HSO₄⁻ (hydrogen sulphate).

In an embodiment, the invention relates to the method of labeling a bacterium as defined above, wherein the modified choline corresponds to the formula (I): 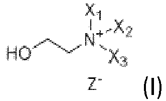 wherein the groups X₁ is an alkene group 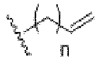 wherein n = 0 to 5, in particular n = 1, 2, 3, 4 or 5,
and X₂ and X₃ are methyl groups 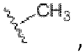 and
Z⁻ is a counterion, preferably selected from F⁻, Cl⁻, Br⁻, I⁻, Tosyl⁻, Mesyl⁻, Triflate⁻, HSO₄⁻ (hydrogen sulphate).

In an embodiment, the invention relates to the method of labeling a bacterium as defined above, wherein the modified choline corresponds to the formula (I): 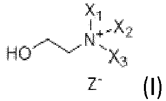 wherein the groups X₁ is an alkyne group 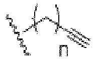 wherein n = 0 to 5, in particular n = 1, 2, 3, 4 or 5,
and X₂ and X₃ are methyl groups 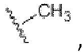 and
Z⁻ is a counterion, preferably selected from F⁻, Cl⁻, Br⁻, I⁻, Tosyl⁻, Mesyl⁻, Triflate⁻, HSO₄⁻ (hydrogen sulphate).

In an embodiment, the invention relates to the method of labeling a bacterium as defined above, wherein the modified choline corresponds to the formula (I): 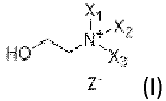 wherein the groups X₁ is an azide group 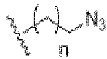 wherein n = 0 to 5,
and X₂ and X₃ are methyl groups 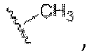 and
Z⁻ is a counterion, preferably selected from F⁻, Cl⁻, Br⁻, I⁻, Tosyl⁻, Mesyl⁻, Triflate⁻, HSO₄⁻ (hydrogen sulphate).

In an embodiment, the invention relates to the method of labeling a bacterium as defined above, wherein the modified choline corresponds to the formula (I): 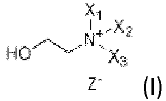 wherein the groups X₁ is a cyclopropenyl group 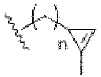 wherein n = 0 to 5, in particular n = 1, 2, 3, 4 or 5,
and X₂ and X₃ are methyl groups 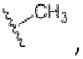 and
Z⁻ is a counterion, preferably selected from F⁻, Cl⁻, Br, I⁻, Tosyl⁻, Mesyl⁻, Triflate⁻, HSO4⁻ (hydrogen sulphate).

In an embodiment, the invention relates to the method of labeling a bacterium as defined above, wherein the modified choline corresponds to the formula (I): 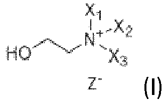 wherein the groups X₁ is a diazirine group 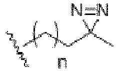 wherein n = 0 to 5, in particular n = 1, 2, 3, 4 or 5,
and X₂ and X₃ are methyl groups 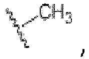 and
Z⁻ is a counterion, preferably selected from F⁻, Cl⁻, Br, I⁻, Tosyl⁻, Mesyl⁻, Triflate⁻, HSO₄⁻ (hydrogen sulphate).

In an embodiment, the invention relates to the method of labeling a bacterium as defined above, wherein the modified choline is selected from the group consisting of:
- Propargyl-choline 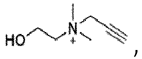
- 1-azidoethyl-choline 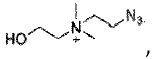
- Allyl-choline 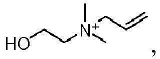
- cyclopropyl-choline 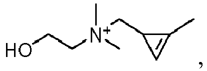
- azidoethyl-diazinebutyl-choline

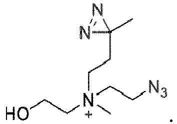

In an embodiment, the invention relates to the method of labeling a bacterium as defined above, wherein the tag molecule is selected from the group consisting of a fluorescent molecule, a luminescent molecule, a radioactive molecule, a biotin molecule or a derivative thereof and an antigen molecule.

In an embodiment, the invention relates to the method of labeling a bacterium as defined above, wherein the tag molecule comprises at least one reactive group Y allowing its binding to the modified choline, said at least one reactive group Y being preferably selected from the group consisting of an alkene group, an alkyne group, an azide group, a cyclopropenyl group, a tetrazine group, a dibenzocyclooctyl (DBCO) group, a dibenzocyclooctine (DIBO) group, a bicyclononine (BCN) group, a Trans-Cyclooctene (TCO) group and a strained Trans-Cyclooctene (sTCO) group.

In an embodiment, the invention relates to the method of labeling a bacterium as defined above, wherein the tag molecule is selected from the group consisting of:
- Cy3 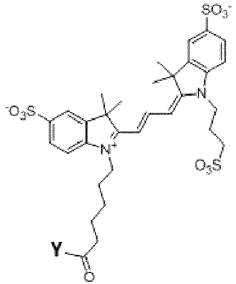
- Cy5 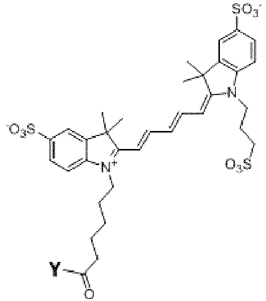
- Alexa 647 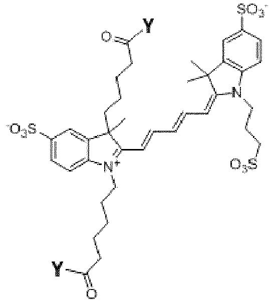
- Fluorescein 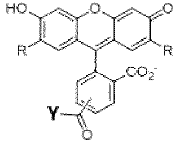 wherein R is H, Alkyl, Aromatic group, OR', NR'₂, SR' (R' = H, alkyl),
- Rhodamine 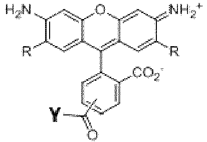 wherein R is H, Alkyl, Aromatic group, OR', NR'₂, SR' (R' = H, alkyl), - Coumarin 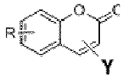 wherein R is H, Alkyl, Aromatic group, OR', NR'₂, SR' (R' = H, alkyl), - BODIPY 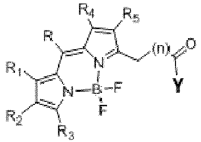 wherein R, R₁, R₂, R₃, R₄ and R₅ are H, Alkyl, Aromatic group, OR', NR'₂, SR' (R' = H, alkyl), - DNP 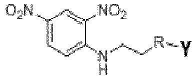 wherein R is Alkyl, Aromatic group, - Biotine 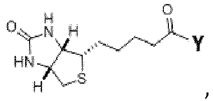 - ATTOS 488 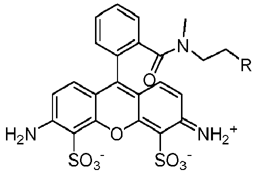 wherein R is Alkyl, Aromatic group,
   wherein alkyl groups are composed from 1 to 10 carbons and aromatic groups are benzenic, indolic, furanyl and pyranyl groups,
   wherein the reactive group Y is selected from the group consisting of:
   - an alkene group 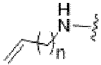 wherein n = 0 to 5, in particular n = 1, 2, 3, 4 or 5,
   - an alkyne group 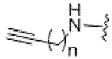 wherein n = 0 to 5, in particular n = 1, 2, 3, 4 or 5,
   - an azide group 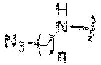 wherein n = 0 to 5, in particular n = 1, 2, 3, 4 or 5,
   - a cyclopropenyl group 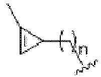 wherein n = 0 to 5, in particular n = 1, 2, 3, 4 or 5,
   - a tetrazine group 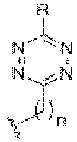 wherein n = 0 to 10, in particular n = 1, 2, 3, 4, 5, 6, 7, 8, 9 or 10,
   - a DBCO group 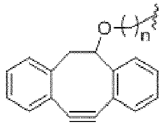 wherein n = 0 to 10, in particular n = 1, 2, 3, 4, 5, 6, 7, 8, 9 or 10,
   - a DIBO group 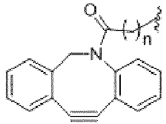 wherein n = 0 to 10, in particular n = 1, 2, 3, 4, 5, 6, 7, 8, 9 or 10,
   - a BCN group 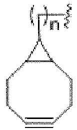 wherein n = 0 to 10, in particular n = 1, 2, 3, 4, 5, 6, 7, 8, 9 or 10,
   - a TCO group 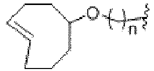 wherein n = 0 to 10, in particular n = 1, 2, 3, 4, 5, 6, 7, 8, 9 or 10,
   - a sTCO group 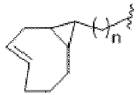 wherein n = 0 to 10, in particular n = 1, 2, 3, 4, 5, 6, 7, 8, 9 or 10.

In an embodiment, the invention relates to the method of labeling a bacterium as defined above, wherein the tag molecule is fluorescent (such as Fluorescein, Rhodamine, Bodipy, ...) and contains a clickable function (such as alkyne, azide, DIBO, tetrazine, ...).

In an embodiment, the invention relates to the method of labeling a bacterium as defined above, wherein the tag molecule is selected from the group consisting of :
- 3-Azido-7-(diethylamino)-2H-chromen-2-one and
- 7-nitro-N-(prop-2-yn-1-yl)benzo[c][1,2,5]oxadiazol-4-amine.

In the invention, at least one reactive group X of the modified choline bound to the TA in the cell wall of the bacterium will react with one reactive group Y of the tag molecule via a bioorthogonal reaction, in particular by a Click chemistry reaction, to form a conjugate and to allow the labeling of the bacterium.

In an embodiment, the invention relates to the method of labeling a bacterium as defined above, wherein the reactive group X of the modified choline and the reactive group Y of tag molecule are respectively selected from the clickable couples recited in Table 1.

**Table 1. Clickable couples of reactive groups X of the modified choline / reactive groups Y of the tag molecule.**

| **Reactive group X (modified choline)** | **Reactive group Y (tag molecule)** |
|---|---|
| Alkene | Tetrazine |
| Alkyne | Azide, Tetrazine |
| Azide | DIBO, DBCO, BCN |
| Cyclopropenyl | Tetrazine |

In the invention, the medium used to incubate the bacterium is selected according to the bacterium to label. The appropriate medium can be selected and/or adapted by one skilled in the art from commercial media or from routinely used media.

In the case of *S*. *pneumoniae,* a C-medium is preferably used. The composition of the C-medium is given in Lacks S , Hotchkiss RD. 1960 (A study of the genetic material determining an enzyme in Pneumococcus. Biochem. Biophys. Acta, 39 :508-518).

For the incubation of the bacterium with the modified choline, the modified choline can be present in the culture medium at various concentrations.

In an embodiment, the invention relates to the method of labelling a bacterium as defined above, wherein, at step (i), the modified choline is present at a concentration of 1 µg/ml to 1 mg/ml, preferably 1 to 100 µg/ml, more preferably 1 to 10 µg/ml, in the culture medium.

As a non-limitative example, the modified choline can be present at a concentration of 1, 2, 3, 4, 5, 6, 7, 8, 9, 10, 20, 50, 100, 200, 500 or 1000 µg/ml in the culture medium. For the labelling step, the bacterium can be incubated in presence of a Copper ligand, such as the THTPA.

The THPTA ligand binds Cu(I), blocking the bioavailability of Cu(I) and ameliorating the potential toxic effects while maintaining the catalytic effectiveness in Click conjugations. The THPTA ligand is used to label living cells with high efficiency while maintaining cell viability.

The time of incubation of the bacterium with the modified choline can vary from few seconds to few hours.

In an embodiment, the invention relates to the method of labelling a bacterium as defined above, wherein, at step (i), the bacterium is incubated with the modified choline for 15 sec to 3 hours or more, preferably for 15 sec to 30 min, even more preferably for 15 to 60 sec.

As a non-limitative example, the time of incubation with the modified choline can be 15 sec, 30 sec, 1 min, 2 min, 10 min, 30 min, 1h, 2h or 3h.

In an embodiment, the invention relates to the method of labelling a bacterium as defined above, wherein, at step (ii), a Copper ligand, such as THTPA, is present in the culture medium.

In an embodiment, the invention relates to the method of labelling a bacterium as defined above, wherein, at step (ii), the bacterium is incubated with the tag molecule for 1 min to 3 hours or more, preferably for 1 min to 30 min, even more preferably for 1 min to 5 min.

As a non-limitative example, the time of incubation with the tag molecule can be 1 min, 2 min, 10 min, 30 min, 1h, 2h or 3h.

In an embodiment, the invention relates to the method of labeling a bacterium as defined above, that further comprises a step (iii) of detection and/or quantification of the bacterium by detecting and/or quantifying the tag molecule bound to the bacterium.

The step of detection and/or quantification of the bacterium can be achieved by various techniques depending on the Tag molecule that has been used for the labeling. These routine techniques (such as epifluorescence, tomography, ...) are well-known by one skilled in the relevant art.

The labeling method of the invention facilitates the detection of the bacterium since it allows a high density labeling, with a grafting level of more than 70%, compared to other labeling techniques such as incorporation of D-amino acids (≈ 2-3%). The grafting level can be determined by various methods well known to the one skilled in the art, such as HPLC analyses (Kuru et al.. ,2012. In situ probing of newly synthesized peptidoglycan in live bacteria with fluorescent D-amino acids. Angew. Chem. Int. Ed. Engl. 51, 12519-12523).

In a preferred embodiment, the invention relates to a method of labeling a bacterium that is able to metabolize choline, preferably *S*. *pneumoniae,* said method comprising:
- a step (i) of incubating the bacterium in a culture medium containing a modified choline during which the modified choline is metabolized by the bacterium and covalently bound to the teichoic acid present in the cell wall of the bacterium,
- a step (ii) of contacting the bacterium with a tag molecule to generate a binding reaction between the modified choline bound to the teichoic acid present in the cell wall of the bacterium and the tag molecule, said binding reaction being preferably made by a Click chemistry reaction, and
- optionally, a step (iii) of detection and/or quantification of the bacterium by detecting and/or quantifying the tag molecule bound to the bacterium.

In a preferred embodiment, the invention relates to a method of labeling a bacterium that is able to metabolize choline, preferably *S*. *pneumoniae,* said method comprising:
- a step (i) of incubating the bacterium in a culture medium containing a modified choline at a concentration of 1 µg/ml to 1 mg/ml, preferably of 1 µg/ml to 10 µg/ml, for 15 sec to 30 min, preferably for 15 to 60 sec, during which the modified choline is metabolized by the bacterium and covalently bound to the teichoic acid present in the cell wall of the bacterium,
- a step (ii) of contacting the bacterium with a tag molecule for 1 min to 30 min, preferably for 1 min to 5 min, to generate a binding reaction between the modified choline bound to the teichoic acid present in the cell wall of the bacterium and the tag molecule, said binding reaction being preferably made by a Click chemistry reaction, and
- optionally, a step (iii) of detection and/or quantification of the bacterium by detecting and/or quantifying the tag molecule bound to the bacterium.

In a preferred protocol, a bacterium *S*. *pneumoniae* is grown, in C-medium in presence of choline, preferably at 6 µg/ml up to an absorbance of 0.3, then the culture is centrifuged and the bacterium is washed with C-medium without choline and concentrated, preferably by a factor 100. Then, 50 µl of the bacterium in suspension is incubated for 15 to 60 sec in C-medium containing 6 µg/ml of azide-choline at 37°C, then 25µM of DIBO-ATTOS 488 is added in the C-medium, then the culture is incubated at 37°C for 5 min. The reaction is stopped by three washes with 1 ml of cold PBS. The bacterium is then re-suspended in 20-40 µl of PBS and observed immediately by fluorescence microscopy.

### Bio-imagery

In another aspect, the invention relates to an *in vitro* method of tracking a bacterium by bio-imagery comprising a step whereby the bacterium is labeled by using the labeling method defined above with a tag molecule allowing the follow-up, preferably in real-time, of the bacterium.

In the field of bio-imaging, the metabolites to be incorporated are all quite expensive to produce (*e.g*. azidio-functionalized L-fucose, KDO sugar, ketone bearing derivatives MurNAc-pentapeptide, GlcNAc precursor, modified tripeptideL-alanyl-g-D-glutamyl-L-Lysine, fluorescent D-amino acids). Thus, the method of the invention has the advantage to be more affordable because the modified cholines can be produced in on-step from 2-(dimethylamino)ethan-1-ol.

For radiolabeling, radiolabeled metabolites like ¹⁸F or ¹⁴C sugar are commonly used but are expensive and complicated to synthesize and to purify. The method of the invention is simpler because it can use Na¹²⁵I as radiolabeling reagent.

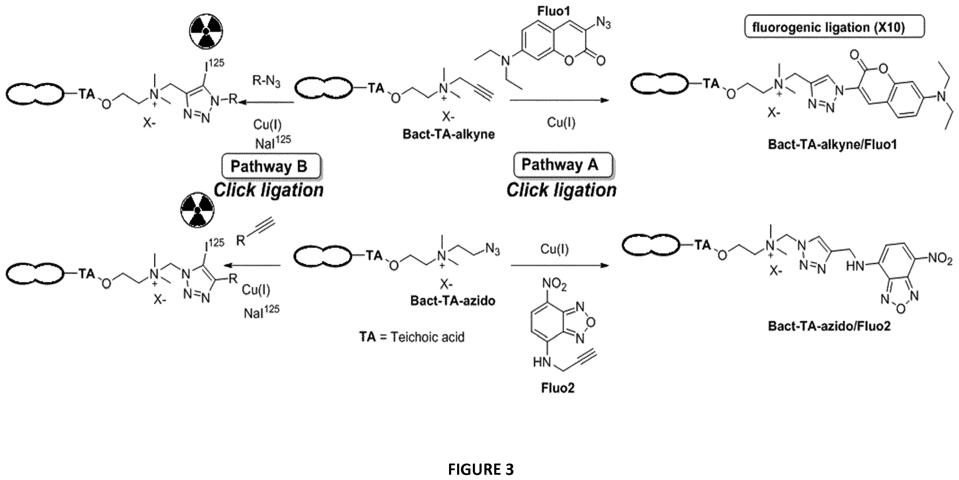

An illustration of the method of tracking is shown in **Figure 3****.**

### Diagnosis

In another aspect, the invention relates to an *in vitro* method for the diagnosis of a bacterial infection from a biological sample of a patient comprising a step whereby the bacterium responsible of the infection is labeled by using the labeling method defined above with a tag molecule allowing the detection of the bacterium.

The method of labeling of the invention can be used to detect the bacteria that are present in a biological sample or the bacteria that have been previously isolated from a biological sample.

The detection of a tagged bacterium is indicative of an infection by said bacterium.

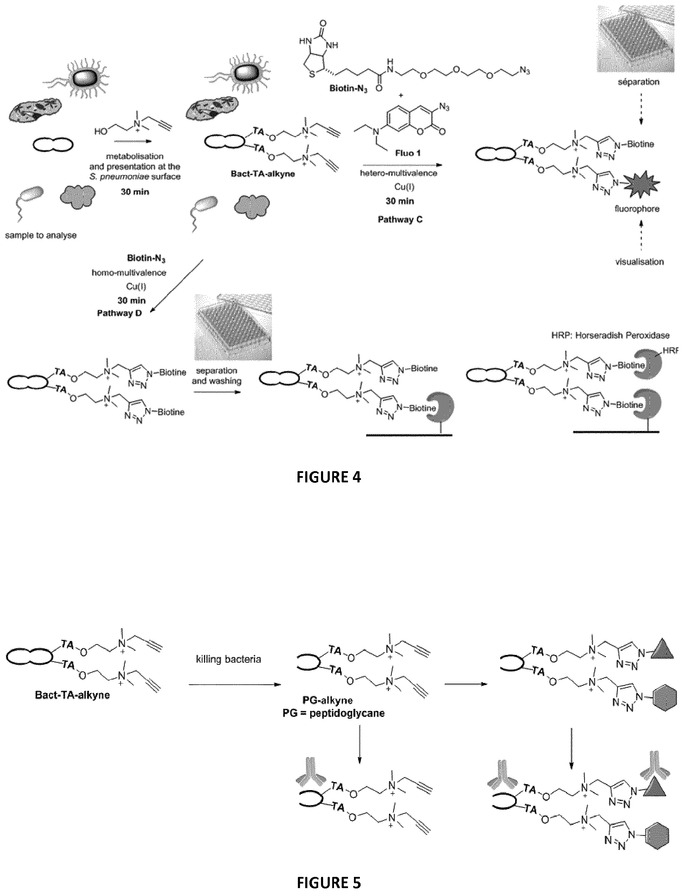

An illustration of the diagnostic method is shown in **Figure 4****.**

### Vaccination

In another aspect, the invention relates to a method for the preparation of a vaccine composition containing a bacterium or fragments thereof, comprising a step whereby the bacterium is *in vitro* labeled by using the labeling method defined above with an antigen, said antigen being bound to the bacterium or to the fragments thereof.

The method of the invention allows to graft a high-number of antigens at the surface of the bacterium and, advantageously, onto the cell wall of the bacterium, which is generally used as a shield by the bacterium to escape or to protect itself from the host immune response. Since the method of labeling allows a high density labeling, this feature is used to turn the bacterium into a multivalent antigen-presenting reagent.

In the invention, the term "antigen" refers to any molecule or compound that induces or enhances an immune response in the host to whom it is administered. In particular, the term antigen covers epitopes that are specifically recognized by antibodies and receptors of the immune agents, such as T-cells, B-cells, NK-cells, ...

In an embodiment, the invention relates to a method for the preparation of a vaccine composition as defined above, which further comprises a step wherein, after labeling, the bacterium is killed, for example by heat treatment or grinding.

In an embodiment, the invention relates to a method for the preparation of a vaccine composition as defined above, which further comprises a step wherein, after labeling, the content of the bacterium is emptied to generate tagged-sacculi, *i.e.* the exoskeleton of the bacterium.

The vaccine composition obtained by the method of the invention can contain live bacteria, dead bacteria, emptied bacteria or fragments of bacteria.

An illustration of the method to prepare a vaccine composition is shown in **Figure 5****.**

### Bio-materials

In another aspect, the invention relates to an *in vitro* method for the preparation of a bio-material comprising:
- a step of labeling a first population of bacteria with a first modified choline by using the labeling method as defined above,
- a step of labeling a second population of bacteria with a second modified choline by using the labeling method as defined above,
the first modified choline used to label the first population and the second modified choline used to label the second population being respectively chosen to cross-react by Click chemistry between each other, and
- then, a step of binding the first population with the second population by Click chemistry to form a bio-material.

In the invention, the bio-material can be assimilated to a prokaryote tissue composed by interconnected bacteria, or fragments of bacteria, linked to each other.

The method of labeling of the invention can thus be used to create bonds between populations of bacteria that have metabolized cross-reacting modified cholines.

In an embodiment, the invention relates to a method for the preparation of a bio-material as defined above that further comprises a step of killing the bacteria, while preserving the cell structure, i.e. the exoskeleton, of the bacteria and the links operated between bacteria.

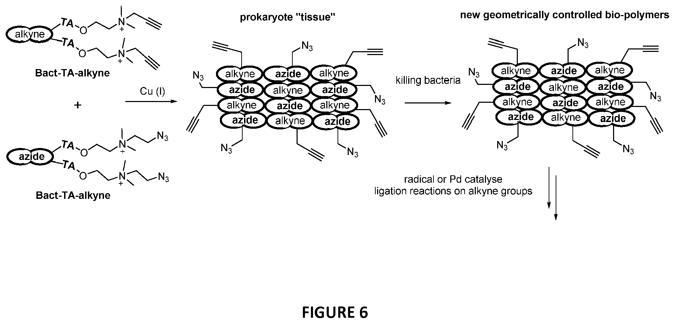

An illustration of the method to produce bio-materials is shown in **Figure 6****.**

### Identification of inhibitors of the cell wall synthesis

In another aspect, the invention relates to an *in vitro* method of identifying an agent that inhibits the bacterial cell wall synthesis, said method comprising:
- a step of contacting a bacterium with a test agent,
- a step of labeling the bacterium using the labeling method defined above,
wherein a test agent that inhibits the labeling of the bacterium is considered a candidate agent for inhibiting the cell wall synthesis of the bacterium.

In particular, this method can be transposed for a high-throughput screening method to screen inhibitors of the peptidoglycane synthesis. The decreased in intensity (e.g. fluorescence) of the signal is correlated to drug susceptibility.

### Kit

In an aspect, the invention relates to a kit to label a bacterium comprising:
- a modified choline as defined above,
- a tag molecule as defined above, and
- a culture medium allowing the growth of the bacterium.

The following figures and examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

**Figure 1****.** Illustration of the structure of TA from *S*. *pneumoniae* (adapted from Gisch et al., J. Biol. Chem., 288:15654, 2013)
**Figure 2****.** Illustration of the Copper-dependent and Copper-independent Click chemistry pathways.
**Figure 3****.** Bacteria coated by alkyne or azide groups are labeled in one step to provide a fluorophore (Pathway A) or a radio-tracer (pathway B).
**Figure 4****.** Illustration of the use of the labeling method of the invention for diagnostic applications.
**Figure 5****.** Illustration of the use of the labeling method of the invention to coat bacteria cell surface with immune-stimulating epitopes.
**Figure 6****.** Illustration of the covalent interlocking of bacteria to form prokaryote tissues and new bio-materials.
**Figure 7****. A.** Metabolization of modified cholines (*S*. *pneumoniae* growth with 10 µg/mL). **B.** Exogenous GFP-LytA displays similar binding pattern on cells grown in presence of propargyl-choline and of normal choline (fluorescent and phase contrast images are shown).
**Figure 8****.** NMR analysis of the propargyl-choline incorporation. Sections of ³¹P NMR (δ_{P} 2.5-(-1.0)) and ¹H NMR (water suppressed; δ_{H} 6.0-0.0)) spectra of PGN-WTA preparations after LytA treatment, isolated from *S*. *pneumoniae* grown in the presence of (i) normal choline (and performed bioorthogonal reaction with 3-azido-7-(diethylamino)-2H-chromen-2-one (2)), (ii) propargyl-choline and (iii) propargyl-choline (and performed bioorthogonal reaction with 2). Incorporation of propargyl-choline in WTA is proven by the occurrence of the additional signals in panels (ii) and (iii).
**Figure 9****.** Detection of metabolically incorporated propargyl-choline in *S*. *pneumoniae* fixed cells before bioorthogonal reaction with coumarin. Numbers (i) to (iii) refer to different stages of division. Phase contrast and fluorescence images in absence **(A)** or presence **(B)** of propargyl-choline are shown. Scale bar = 1 µm

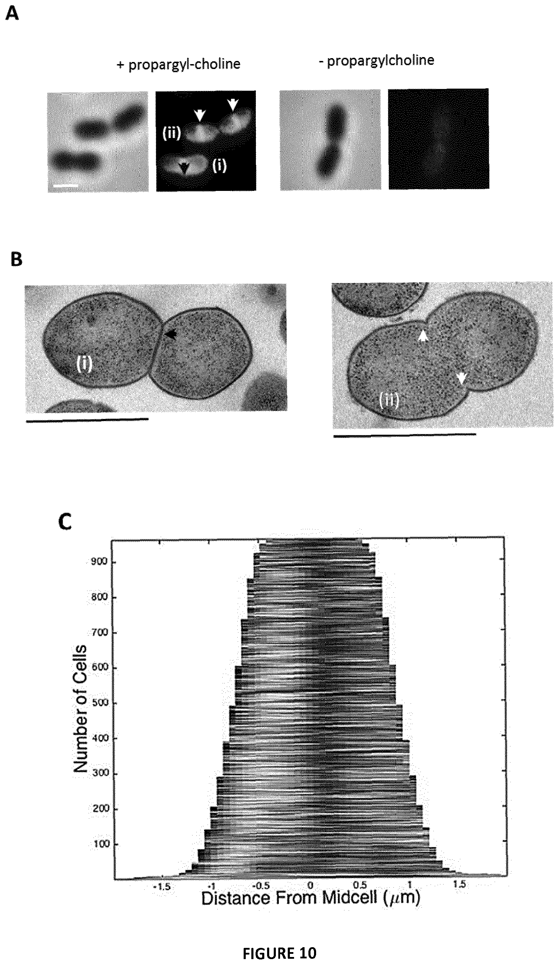

**Figure 10****.** TA metabolic labeling on pneumococcal live cells. **A.** Detection of metabolically incorporated propargyl-choline in *S*. *pneumoniae* living cells after bioorthogonal reaction. (i) and (ii) refer to two different stages of division. Membrane and septal TA labeling are indicated by black and white arrow heads, respectively. Phase contrast and fluorescence images in the presence (left panel) or absence of propargyl-choline (right panel) are shown. Scale bar = 1 µm. **B.** Electron micrographs of pneumococcal cells at division stages (i) and (ii) as shown in A. **C.** Demograph of a pneucococcal cell population grown in the presence of propargyl-choline and submitted to the bioorthogonal reaction shows the mid-cell positioning of TA.
**Figure 11****.** TA metabolic labeling on live cells after 30-min pulse of propargyl-choline. **A.** Numbers (i) to (iii) refer to the different stages of division. Phase contrast and fluorescence images are shown. Scale bar = 1 µm. **B.** Demograph of pneucococcal cell population grown in the presence of propargyl-choline for 30 min and labeled with propargyl-choline-coumarin showing the mid-cell positioning of TA.
**Figure 12****.** Specific detection of metabolically incorporated propargyl-choline in live *S*. *pneumoniae* cells. Cultures of *E. coli, B. subtilis* and *P. aeruginosa* were performed as for *S*. *pneumoniae* in the presence (+ propargyl-Cho) or in the absence (+ Cho) of propargyl-choline. Cultures were mixed as indicated on the left hand side of the figure and processed for biorthogonal reaction.
**Figure 13****.** Short pulse. The bacterium is incubated for 15 seconds (left) or 60 seconds (right) with the the azide-choline, and then incubated for 5 minutes with DIBO-ATTOS 488.

### EXAMPLES

### Materials and Methods

### Bacterial growth conditions

Liquid cultures of the unencapsulated pneumococcal strain R6 were grown at 37°C - 5%CO₂ in a chemically defined medium (C-medium) supplemented with 4 µg/ml choline (Cmed-choline). Contrary to the original composition, the C-medium used here did not contain neither yeast extract nor albumin. Cells were harvested by centrifugation at 3,320 g for 10 min, washed three times with C-medium without choline, concentrated to OD₆₀₀ₙₘ of 2 and stored at -80°C as aliquots containing 15% glycerol (v/v).

For bioorthogonal reactions, 10 ml of Cmed-choline and 10 ml of C-medium containing 4 µg/ml of propargyl-choline were inoculated at OD₆₀₀ₙₘ of 0.05 with aliquots of cells conditioned in C-medium as described above. The growth was pursued at 37°C - 5%CO₂ for 3 hours until OD₆₀₀ₙₘ of 0.2-0.25 was reached, which corresponds to the early exponential growth phase. The cells were pelleted by centrifugation at 3,320 g for 10 min and subsequently incubated with 500 µl of 2% choline chloride (w/v) for 10 min at room temperature (RT) to remove the Choline-Binding Proteins (CBPs) that bind to choline residues. In the case of choline-alkyl residues, the presence of CBPs possibly impair the labeling of those molecules by the fluorescent azide reporter. The cells were washed twice with PBS (1 min centrifugation at 4,500 g) and resuspended in 400 µl of PBS. A volume of 100 µl was used for each bioorthogonal reaction. In pulse experiments, cells were grown in Cmed-choline for 3h, washed twice in PBS, resuspended in C-medium containing 4 µg/ml of propargylcholine or 1-azidoethyl-choline and incubated at 37°C - 5%CO₂ for 30 min before proceeding to the labeling.

*Escherichia coli, Bacillus subtilis* and *P. aeruginosa* growth conditions in C-medium supplemented with both forms of choline were tested before conducting the click reactions with the same protocol as the one developed for *Streptococcus pneumoniae.*

### Copper catalyzed click chemistry

Labeling was performed on cells grown in presence of choline and choline-alkyl. A volume of 100 µl of cell suspension prepared as described above was incubated with the following reagents, which final concentration is indicated: coumarin (1 mM), ascorbic acid (1 mM), Copper (II) sulfate (50 µM), THPTA (tris(3-hydroxypropyltriazolylmethyl)amine) (300 µM) for 30 min at RT, under mild agitation and protected from the light. Labeled cells were washed twice with PBS and resuspended in PBS for microscopy observation.

Cell fixation was performed after culture harvest. Cells from 10 ml culture were washed twice with PBS, resuspended in 500 µl of 4% (w/v) paraformaldehyde for 30 min at RT followed by a 2 h-incubation at 4°C. After two washes with PBS, cells were resuspended in 400 µl of PBS and aliquots of 100 µl were used for the click reaction by adding the coumarin (1 mM), ascorbic acid (1 mM) and Copper (II) sulfate (100 µM). The labeling proceeded for 16 h at RT under mild agitation and protected from the light. Labeled cells were washed twice with PBS and resuspended in PBS before microscopy observation.

### Fluorescence microscopy and image analysis

Pneumococcal cells were transferred to microscope slides and observed using an Olympus BX61 optical microscope equipped with a UPFLN 100x O-2PH/1.3 objective and a QImaging Retiga-SRV 1394 cooled charge-coupled device camera. Image acquisition and analysis were performed using the software packages Volocity and open-source Oufti, respectively and processed with Adobe Photoshop CS5. Cell population demographs were constructed by Oufti which integrates the signal values in each cell. The cells are then sorted by their length value and the fluorescence values are plotted as a heat map.

### Extraction and isolation of LTA

Pneumococcal cells were resuspended in citrate buffer (50 mM, pH 4.7) and disrupted three times by French press (Constant Cell Disruption System, Serial No. 1020) at 10 °C at a pressure of 20 kPSI. SDS was added to a final concentration of 4% to the combined supernatants. The solution was incubated for 30 min at 100 °C and was stirred afterwards overnight at room temperature. The solution was centrifuged at 30,000 x g for 15 min at 4 °C. The pellet was washed four times with citrate buffer using the centrifugation conditions as above. The combined LTA-containing supernatants and the resulting sediment, containing the crude PGN-WTA complex, were lyophilized separately. The resulting solids were both washed five times with ethanol (centrifugation: 20 min, 20 °C, 10,650 x g) to remove SDS and lyophilized (leading to pellet A containing LTA and pellet B containing the PGN-WTA complex). For LTA isolation, pellet A was resuspended in citrate buffer and extracted with an equal volume of butan-1-ol (Merck) at room temperature under vigorous stirring. The phases were separated by centrifugation at 4,000 x g for 15 min at 4 °C. The aqueous phase (containing LTA) was collected, and the extraction procedure was repeated twice with the organic phase plus interphase. The combined aqueous phases were lyophilized and subsequently dialyzed for 5 days at 4 °C against 50 mM ammonium acetate buffer (pH 4.7; 3.5 kDa cut-off membrane); the buffer was changed every 24 h. The resulting crude LTA was purified further by hydrophobic interaction chromatography (HIC) performed on a HiPrep Octyl-Sepharose column (GE Healthcare; 16 x 100 mm, bed volume 20 ml). The crude LTA material was dissolved in as little starting buffer (15% propan-1-ol (Roth) in 0.1 M ammonium acetate (pH 4.7)) as possible and centrifuged at 13,000 x g for 5 min at room temperature and the resulting supernatant was lyophilized. The LTA-containing pellet was dissolved in the HIC start buffer at a concentration of 30 mg/ml and purified by HIC using a linear gradient from 15% to 60% propan-1-ol (Roth) in 0.1 M ammonium acetate (pH 4.7). LTA-containing fractions were identified by a photometric phosphate test. The phosphate-containing fractions were combined, lyophilized and washed with water upon freeze-drying to remove residual buffer.

### Extraction and isolation of WTA

Pellet B (containing the crude PGN-WTA complex), which arose during LTA isolation, was resuspended at a concentration of 10 mg/ml in 100 mM Tris-HCl (pH 7.5) containing 20 mM MgSO₄. DNase A and RNase I were added to final concentrations of 10 and 50 µg/ml, respectively. The suspension was stirred for 2 h at 37 °C. Subsequently, 10 mM CaCl₂ and trypsin (100 µg/ml) were added and the stirring was continued overnight at 37 °C. SDS at a final concentration of 1% was added, and the mixture was incubated for 15 min at 80 °C to inactivate the enzymes. The cell wall was recovered by centrifugation for 45 min at 130,000 x g at 37 °C. The resulting pellet was resuspended in 0.8 ml 8 M LiCl per 1 ml initially used Tris-HCl solution and incubated for 15 min at 37 °C. After another centrifugation using the same conditions as above, the pellet was resuspended in 1 ml 10 mM ethylenediaminetetraacetic acid (EDTA, pH 7.0) per ml of the Tris-HCl solution used initially and this sample was incubated at 37 °C for 15 min. The pellet was washed twice with water. Finally, the pellet was resuspended in 2 to 4 ml of water and lyophilized, yielding the purified PGN-WTA complex. To remove all amino acids from the PGN, the PGN-WTA complex was dissolved in 50 mM Tris-HCl (pH 7.0; 10 mg/ml) and treated with the pneumococcal LytA amidase. Recombinant His-tagged LytA amidase (1 mg / 10 µg LytA) was added in three aliquots after 0, 24 and 48 h for a total period of incubation of 72 h at 37 °C. Subsequently, the enzyme was inactivated by boiling for 5 min at 100 °C. After centrifugation (25,000 x g, 15 min, 20 °C) the supernatant was collected and lyophilized. The crude LytA-treated PGN-WTA complex was further purified by GPC on a Bio-Gel P-30 (45-90 µm, BioRad; column size: 1.5 x 120 cm; buffer: 150 mM ammonium acetate (pH 4.7)) column.

### Example 1. Metabolic incorporation of modified choline

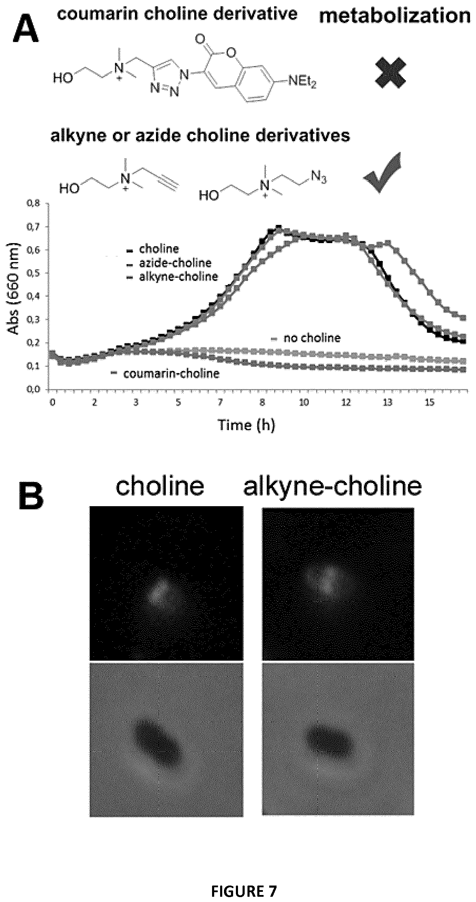

The choline dependency for pneumococcal growth was exploited to validate the metabolic incorporation of modified cholines. Propargyl-choline was evaluated as well as its corresponding fluorescent analogue (i.e. propargyl-choline-coumarin) obtained by the click reaction between propargyl-choline and coumarin. One interesting aspect of the coumarin comes from its fluorogenic property once coupled with alkyne that amplifies the fluorescence signal to reduce the background interference. Nonpathogenic (unencapsulated) pneumococcal R6 strain was cultured in C-medium containing propargyl-choline-coumarin or normal choline. Comparable growth rates were observed in the presence of normal choline and of propargyl-choline indicating a good metabolisation of the latter compound **(****Figure 7****).**

### Example 2. Analytical characterization and quantification of TA decorated by modified choline

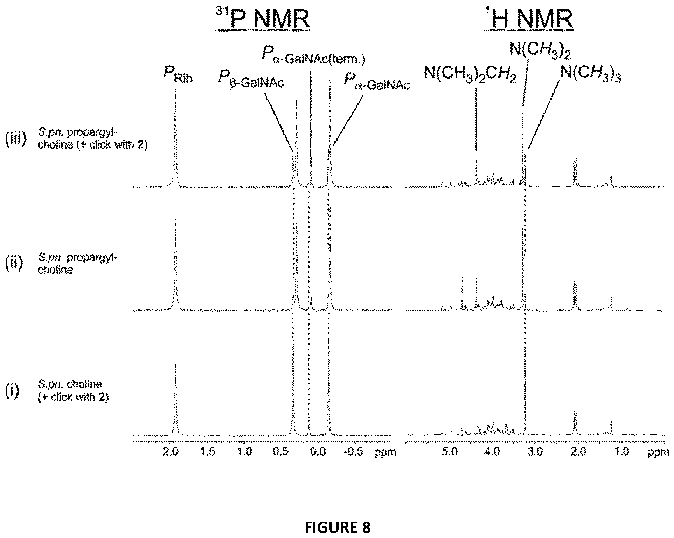

Pneumococcal cultures grown in presence of propargyl-choline or normal choline were processed to extract LTA. NMR analysis showed that propargyl-choline has been integrated in LTA **(****Figure 8****).**

### Example 3. Detection of metabolically incorporated modified choline

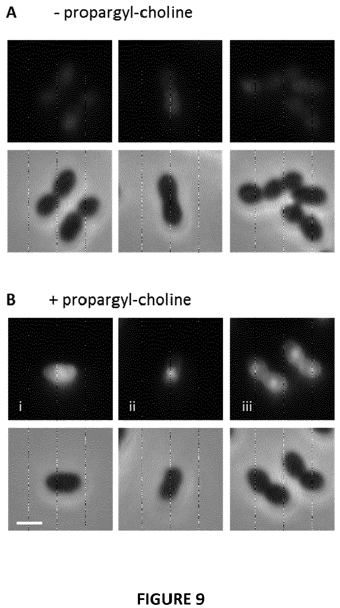

To prevent any modification of the structure of TA and/or their eventual re-localization at the cell surface during the chemical labeling, a preliminary study was performed with cell containing propargyl-choline fixed prior the bioorthogonal reaction. Fluorescence was specifically detected on cells grown in the presence of propargyl-choline when compared with cells grown in the presence of normal choline **(****Figure 9****).** Bright fluorescent spots were observed at the mid-cell position in early (panels i and ii) and late division stages (panel iii).

### Example 4. Metabolic labeling on pneumococcal live cells

To obtain information on the TA biosynthesis dynamics, labeling of live pneumococcal cells have been performed after optimizing the reactional conditions. Reduction of copper concentration to 50 µM, addition of the catalyst THPTA and reducting the incubation time to 30 min allowed to specifically label TA **(****Figure 10A****).** The fluorescence intensity displayed by pneumococcal cells grown in presence of propargyl-choline was measured and compared to the signal detected on cells grown with choline in five independent experiments (n = 155 to 2445 in each experiment and for each culture condition). The fluorescent signal ratio propargyl-choline / choline was 4.28 ± 0.8.

Fluorescence detected at the cell periphery and at the contact zone between daughter cells before they separate suggest a membrane localization of TA/LTA **(****Figure 10A****,** stage (i), black arrow head). An electron micrograph of a pneumococcal cell of similar morphology is shown to appreciate in details the membrane topology **(****Figures 10B****,** stage (i), black arrow head). Early-division stage (ii) is characterized by the on-set of cross-wall synthesis and membrane invagination **(****Figures 10A and 10B****,** white arrow heads). TA labeling is observed at the septal site in these cells indicating that TA are synthesized and/or flipped across the membrane at the same time and the same place as peptidoglycan synthesis. Image analysis of a cell population confirms the septal localization of the TA labeling on pneumococcal cells over the cell cycle **(****Figure 10C****):** after integration of the fluorescence signal in each cell, cells were sorted by their length value and the fluorescence values was plotted as a heat map.

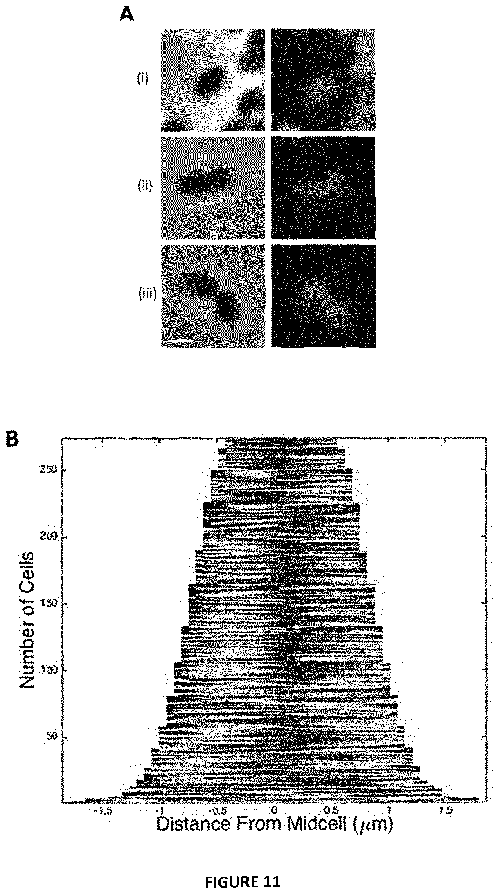

A pulse of propargyl-choline was performed. Pneumococcal cells were grown in medium containing choline, washed, incubated in presence of propargyl-choline for 30 min and submitted to the bioorthogonal reaction **(****Figure 11****).** In these conditions, only TA synthesized during the 30 min pulse are labeled. Reduced membrane labeling (when compared to the 3 h culture period shown in **Figure 8****)** together with the septal site localization **(Figure 11A and** **11B****)** confirm that TA synthesis takes place in a relatively short time scale.

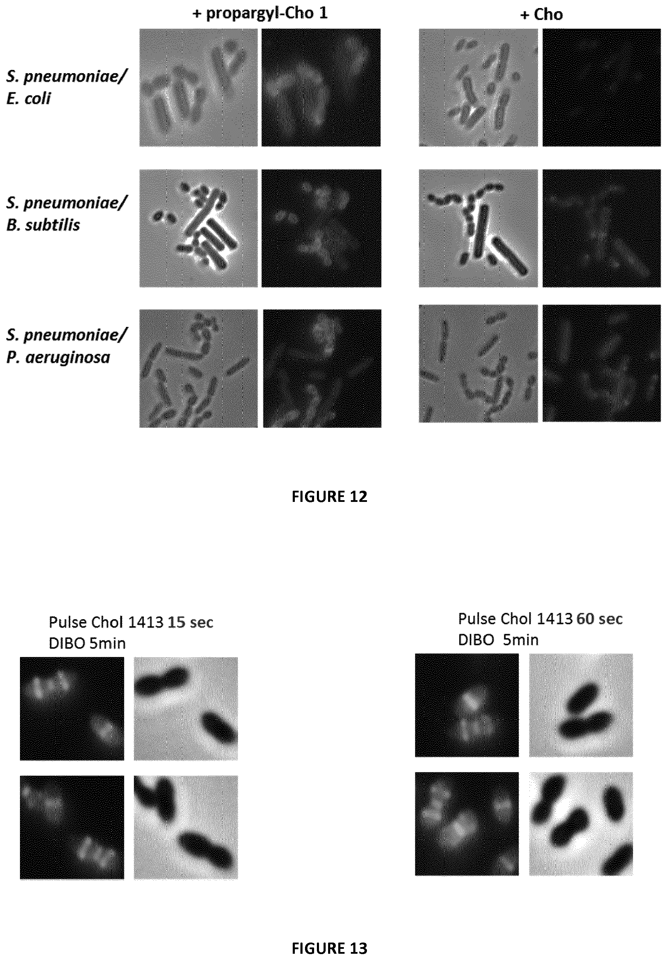

No fluorescent signal was detected when the bioorthogonal reaction was performed on *Escherichia* coli, *Bacillus subtilis* and *Pseudomonas aeruginosa* species, respectively Gram-negative and Gram-positive bacteria that do not metabolize choline **(****Figure 12****).** This work is the first report of metabolic labeling of Gram-positive TA. These results demonstrate the specificity and selectivity of the labeling based on click chemistry. Long and short labeling pulses with propargyl-choline label TA in live cells at the septal site show that TA synthesis might be correlated to the peptidoglycan synthesis and the cell division. The success of this method offers possibility to explore mechanistic issues of pneumococcal TA biosynthesis in a more physiological context.

### Example 5. High-speed labeling of S. pneumoniae

A quick incubation of 15 seconds with the 1-azidoethyl-choline, followed by the addition of DIBO-ATTOS 488 for 5 min, is enough to obtain a high-quality labeling **(****Figure 13****).**

## Claims

1. A method of labeling a bacterium that is able to metabolize choline, said method comprising a step (i) of incubating the bacterium in a culture medium containing a modified choline which is metabolized by the bacterium, covalently associated to the TA into the cytoplasm, before being exported and integrated into the cell wall of the bacterium.

2. The method according to claim 1, wherein said bacterium is is a Gram-positive bacterium, which can be selected from the *Streptococcus* genus, or a Gram-negative bacterium, which can be selected from *Haemophilus* or *Neisseria* genera, and in particular from *S*. *pneumoniae, H. influenzae* and *Neisseria ssp..*

3. The method according to claims 1 or 2, wherein the modified choline is chemically modified to incorporate a radioactive isotope.

4. The method according to claims 1 or 2, that further comprises a step (ii) of contacting the bacterium with a tag molecule to generate a binding reaction between the modified choline bound to the teichoic acid present in the cell wall of the bacterium and the tag molecule, said binding reaction being preferably made by a Click chemistry reaction.

5. The method according to claim 4, wherein the modified choline comprises at least one reactive group X allowing the binding of the modified choline to the tag molecule, said at least one reactive group X being selected from the reactive groups consisting of an alkene group, an alkyne group, an azide group, a cyclopropenyl group and a diazirine group.

6. The method according to claims 4 or 5, wherein the modified choline is selected from the group consisting of:
- Propargyl-choline,
- 1-azidoethyl-choline,
- Allyl-choline,
- cyclopropyl-choline,
- azidoethyl-diazinebutyl-choline.

7. The method according to any one of claims 4 to 6, wherein the tag molecule is selected from the group consisting of a fluorescent molecule, a luminescent molecule, a radioactive molecule, a biotin molecule or a derivative thereof and an antigen molecule.

8. The method according to any one of claims 4 to 7, wherein the tag molecule comprises at least one reactive group Y allowing its binding to the modified choline, said at least one reactive group Y being preferably selected from the group consisting of an alkene group, an alkyne group, an azide group, a cyclopropenyl group, a tetrazine group, a dibenzocyclooctyl (DBCO) group, a dibenzocyclooctine (DIBO) group, a bicyclononine (BCN) group, a Trans-Cyclooctene (TCO) group, a strained Trans-Cyclooctene (sTCO) group.

9. The method according to any one of claims 4 to 8, wherein the tag molecule is selected from the group consisting of :
- 3-Azido-7-(diethylamino)-2H-chromen-2-one and
- 7-nitro-N-(prop-2-yn-1-yl)benzo[c][1,2,5]oxadiazol-4-amine.

10. The method according to any one of claims 4 to 9, that further comprises a step (iii) of detection and/or quantification of the bacterium by detecting and/or quantifying the tag molecule bound to the bacterium.

11. An *in vitro* method of tracking a bacterium by bio-imagery comprising a step whereby the bacterium is labeled, by using the labeling method as defined in any one of claims 1 to 10, with a tag molecule allowing the follow-up, preferably in real-time, of the bacterium.

12. An *in vitro* method for the diagnosis of a bacterial infection from a biological sample of a patient comprising a step whereby the bacterium responsible of the infection is labeled, by using the labeling method as defined in any one of claims 1 to 10, with a tag molecule allowing the detection of the bacterium.

13. A method for the preparation of a vaccine composition containing a bacterium or fragments thereof, comprising a step whereby the bacterium is *in vitro* labeled, by using the labeling method defined in any one of claims 4 to 10, with an antigen, said antigen being bound to the bacterium or to the fragments thereof.

14. An *in vitro* method for the preparation of a bio-material comprising:
- a step of labeling a first population of bacteria with a first modified choline by using the labeling method as defined in any one of claims 4 to 9,
- a step of labeling a second population of bacteria with a second modified choline by using the labeling method as defined in any one of claims 4 to 9,
the first modified choline used to label the first population and the second modified choline used to label the second population being respectively chosen to cross-react by Click chemistry between each other, and
- then, a step of binding the first population with the second population by click chemistry to form a bio-material.

15. An *in vitro* method of identifying an agent that inhibits the bacterial cell wall synthesis, said method comprising:
- a step of contacting a bacterium with a test agent,
- a step of labeling the bacterium using the method as defined in any one of claims 4 to 10,
wherein a test agent that inhibits the labeling of the bacterium is considered a candidate agent for inhibiting the cell wall synthesis of the bacterium.

16. A kit to label a bacterium comprising:
- a modified choline as defined in claims 5 or 6,
- a tag molecule as defined in claims 7 to 9, and
- a culture medium allowing the growth of the bacterium.
